# EUROPEAN PATENT SPECIFICATION

(11) **EP 1 392 249 B1**
(45) Date of publication and mention of the grant of the patent: **02.12.2009**
(21) Application number: 02730465.8
(22) Date of filing: 30.05.2002
(51) Int. Cl.: A61K 9/20, A61K 9/16, A61K 31/192, A61P 29/00

(54) **NSAID FORMULATION COMPRISING A GRANULAR COMPOSITION AND AN EXTRA-GRANULAR COMPOSITION**
NSAID-FORMULIERUNG ENTHALTEND EINE GRANULÖSE ZUSAMMENSETZUNG UND EINE EXTRAGRANULÖSE ZUSAMMENSETZUNG
PREPARATION ANTI-INFLAMMATOIRE NON STEROIDIENNE RENFERMANT UNE COMPOSITION GRANULAIRE ET UNE COMPOSITION EXTRA-GRANULAIRE

(30) Priority: 07.06.2001 GB 0113839
(43) Date of publication of application: 03.03.2004
(73) Proprietor: Reckitt Benckiser Healthcare (UK) Limited, Slough, Berkshire SL1 3UH (GB)
(72) Inventor: RHOADES, Tracey, Jane, The Boots Company PLC, Nottingham NG2 3AA (GB); SHERRY, Robert, Arthur, The Boots Company PLC, Nottingham NG2 3AA (GB)
(74) Representative: O'Brien, Niall James
(86) International application number: PCT/GB2002/002536
(87) International publication number: WO 2002/098392

(56) References cited:
- EP-A- 0 305 356
- EP-B- 0 582 380
- WO-A-01/41733
- US-A- 5 512 300
- US-A- 5 667 807
- US-A- 5 869 101

## Description

This invention relates to compositions containing a non-steroidal anti-inflammatory drug, to processes to prepare them and to uses thereof.

Non-steroidal anti-inflammatory drugs (NSAIDs) are a widely used class of medicaments. They are a well defined group of compounds and include phenylpropionic acids such as ibuprofen, naproxen, ketoprofen and flurbiprofen. They are primarily used for the treatment of one or more of pain, inflammation and fever, for example rheumatoid arthritis, ankylosing spondylitis, osteoarthritis, postoperative pain, post-partum pain and soft tissue injuries. One example is ibuprofen, which is available under prescription in the UK (eg Brufen (RTM)), generally at doses up to 3200 mg per day. Ibuprofen is also available as a non-prescription drug in the UK (eg Nurofen (RTM)) primarily for the treatment of symptoms of pain and fever including headache, migraine, rheumatic pain, muscular pain, backache, neuralgia, dysmenorrhoea, dental pain and colds and flu, generally at doses up to 1200 mg per day.

Ibuprofen and other NSAIDs are generally acidic and substantially insoluble drugs. They are conveniently administered as an oral pharmaceutical composition in the form of tablets. Thus pharmaceutically acceptable excipients must be chosen for combination with the NSAID, with which the NSAID is compatible and with which it can form tablets having a satisfactory hardness and also release the medicament rapidly into the body so that it is available for absorption.

A major issue in connection with the disorders identified above is to improve the onset of action of the NSAID, particularly in the treatment of pain. It is believed that rapid disintegration of a formulation releases the drug into the body quickly leading to a more rapid onset of therapeutic action compared with a standard dosage form. Accordingly, it is desired to produce a solid dosage form for oral administration adapted to disintegrate quickly in the gastro-intestinal tract. Many of the NSAIDs are acidic drugs, accordingly, absorption can be a problem in the acidic conditions encountered in the stomach. Furthermore, although the literature has proposed many formulations adapted to disintegrate quickly, a major problem occurs with ibuprofen and other NSAIDs as they can be administered in relatively high doses, eg up to 800 mg per unit dose. Thus, there is a problem to provide a dosage form which includes the NSAID together with excipients useful to formulate the tablet into the dosage form and also excipients useful to ensure rapid disintegration, but not to provide a tablet that is too large for patient consumption or cannot be produced according to standard large scale manufacturing processes. Furthermore, the solid dosage form must be sufficiently hard to withstand the rigours of the manufacturing process (for example as encountered during the stage of film coating in a perforated rotating drum and packaging etc) but must have appropriate disintegration characteristics to ensure rapid release of the drug from the formulation and also appropriate dissolution characteristics. Another significant problem that must be overcome is to ensure that the composition is capable of being compressed without sticking to the punches of the tabletting machine.

As an alternative to the general path of choosing particular excipients and tabletting conditions or changing the form of the unit dose, one avenue for investigation is to consider modifying the crystalline form of the NSAID in order to try to optimise its performance.

Earlier patent applications have considered heating ibuprofen, a relatively low melting drug, until molten and cooling to form a granulated composition, combining with optional tabletting excipients and compressing into a tablet. Japanese Patent Kokai 81/120616 (1981) describes a process to prepare ibuprofen granules which allows the formation of smaller dosage forms, together with better flow properties of the granulate material prior to tabletting. In the illustrative examples of JP 81/120616, the ibuprofen is melted by heating and excipients such as fine crystalline cellulose and calcium stearate are added (optionally with hydroxypropyl starch) to form a dispersion of the insoluble ingredients within the molten ibuprofen. The mixture is then cooled and crushed to form granules. The granules are either directly compressed into tablets without the addition of further excipients or mixed with Aerosil (colloidal silicon dioxide) and filled into capsules. However, it was shown by measurements of blood concentration that although smaller dosage forms and better flowability were achieved, there was no significant difference in the bioavailability between tablets prepared as described in JPA 81/120616 and those of the art available before 1981.

European Patent Application 362728 (1990) relates to an easily flowable ibuprofen granular composition that has improved storage and formulation properties for direct tabletting. The molten ibuprofen is solidified on a contact cooling apparatus using a seeding process and is then comminuted into a solid. The granulate formed consists wholly of ibuprofen. The process described requires the molten ibuprofen to be rapidly congealed under specific conditions and then seeded when the molten ibuprofen solidifies, the resulting flakes being crushed under specific milling conditions. The illustrative examples describe taking granules formed by this process and combining them with a significant amount of necessary tabletting excipients such as microcrystalline cellulose, sodium starch glycolate, colloidal silicon dioxide and magnesium stearate and compressing into tablets. In order to reduce the size of the tablet it is desired to reduce the quantity of extra-granular excipients necessary for combination with the ibuprofen granules prior to tabletting.

US Patent 5240712 (1993) discloses that molten ibuprofen may be poured into capsules and gives examples of encapsulated dosage forms containing ibuprofen, optionally containing excipients as a solid solution or dispersion therein. The molten ibuprofen composition is filled into a capsule and then allowed to solidify. The dosage forms thus produced need no further processing and can be directly administered to a patient. However, the capsules are of significant size and it is desired to produce a solid dosage form of relatively small size.

US 5667807 (1997) also relates to heating ibuprofen until molten and producing tablets from the granular composition obtained therefrom. It contains illustrative examples of tablets produced firstly by forming a mixture of ibuprofen with excipients (including microcrystalline cellulose, maize starch, magnesium stearate and optionally colloidal silicon dioxide and croscarmellose sodium), and then heating and extruding said mixture in a melt extruder to produce an extrudate in which a part of the active is melted. It is said that the low melting point active fulfils the function of a binder or of a solid solvent, In a second part of the process, the cooled, comminuted granules prepared from the extrudate are compressed into tablets after the optional inclusion of a lubricant. However, it is desired to reduce the number of excipients needing to be incorporated into the composition and to improve the performance of the tablet.

Thus, in formulating a dosage form with granules produced by solidifying molten ibuprofen, it has previously been proposed that either (a) a significant number of excipients are added to the molten ibuprofen and the granules are taken for direct compression into tablets or (b) granules containing only ibuprofen are combined with a significant amount of additional tabletting excipients then compressed into tablets.

EP 0 305 356 discloses a granular product which consists of a solid that is provided with a coating of a melt of said solid. In a preferred embodiment ibuprofen in a melted form is applied to particulate ibuprofen.

US 5 869 101 discloses a process for the production of S(+) - ibuprofen particles which have improved flow properties.

Co-pending PCT application PCT/EP 00/12193 discloses that valuable tablet properties may be obtained by incorporating silicon dioxide into a compressed tablet composition comprising a granular component comprising a plurality of solidified melt granules of a low-melting NSAID incorporating a disintegrant uniformly dispersed therein.

We have now found that valuable tabletting properties can be obtained without being restricted to one key ingredient, namely silicon dioxide. This may have advantages in terms of supply of raw materials, processing of materials and cost. We have found that if a granular composition comprising a plurality of solidified melt granules of a low-melting NSAID, preferably incorporating a disintegrant uniformly dispersed therein is combined with an extra-granular composition comprising an insoluble wicking agent comprising an inorganic material and/or a starch material, wherein the wicking agent is not silicon dioxide, a silica-free NSAID tablet with valuable tabletting properties is produced.

Accordingly, the present invention provides a compressed tablet formulation comprising a granular composition comprising a plurality of solidified melt granules comprising a continuous phase of a low-melting non-steroidal anti-inflammatory drug optionally incorporating a disintegrant uniformly dispersed therein,
**characterised in that** the formulation comprises an extra-granular composition comprising an insoluble wicking agent selected from an inorganic material and a starch material or a mixture thereof,
provided that the formulation does not contain silicon dioxide.

It has been found that formulations prepared according to the present invention have valuable disintegrating properties. Furthermore, the dissolution results show an unexpectedly high level of the NSAID dissolved in the aqueous medium after relatively short periods of time.

A further advantage of the present invention lies in the small amount of additional tabletting excipients needed to prepare a dosage form, thus leading to advantages in processing and cost of the tablets and allowing smaller dosage forms to be produced. Furthermore, the formulation formed prior to tabletting has good flow properties and the resulting tablets have a good hardness.

The surface area of the NSAID in the melt granule is significantly greater than that of conventional crystals of the NSAID. In addition, the particle size is less than the particle size produced by micronising NSAID particles which is a conventionally favoured method of an improving the dissolution. It is surprising that the effect of the small amounts of an insoluble wicking agent has the effect of causing the formulation to disperse so quickly in aqueous conditions, especially in acidic conditions (such as are found in the stomach) leading to a high percentage of NSAID being dissolved within a relatively short period.

The NSAID may be in the form of a racemic mixture or an enantiomer. Pharmaceutically acceptable salts of the NSAID may also be used. Preferred salts are the sodium, potassium or lysine salts of the NSAID.

The invention allows the formulation of any relatively low melting NSAID into an acceptably tasting, readily disintegrating formulation. A favoured class of compounds are the 2-arylpropionic acids which are generally substantially insoluble and have poor taste properties. It is generally envisaged that the melting point of such compounds will be low enough to allow the melting thereof using standard equipment. It is also important that there is not a deleterious effect on any ingredients incorporated in the molten NSAID, for example a disintegrant. Thus, typical melting points of the low melting NSAIDs would be expected to fall within the range 30-300°C. Preferred NSAIDs have lower melting points so that the melting process does not use significant amounts of energy, which thus has an effect on production costs. Preferred melting points are in the range 30-200°C (such as racemic naproxen, melting point 156°C), more preferably 30-150°C, further preferably 40-120°C (such as racemic flurbiprofen, melting point 114°C), most preferably 50-100°C (such as racemic ibuprofen (melting point 75-77°C), S(+)-ibuprofen (melting point 52-54°C) and racemic ketoprofen (melting point 96°C)). Preferred low-melting NSAIDs are naproxen, ketoprofen, flurbiprofen, ibuprofen or enantiomers (especially the S(+)-enantiomers) thereof. The invention is especially adapted for an ibuprofen medicament. The term "ibuprofen medicament" includes racemic ibuprofen and S(+)-ibuprofen which have low melting points and a very poor after-taste in the mouth and throat. Most advantageous results are obtained with racemic ibuprofen which has a high dosage combined with poor solubility properties.

The proportion of NSAID in the granular composition will depend on the dose desired for therapeutic effect. Low dose drugs, such as flurbiprofen and ketoprofen may form as little as 20% by weight (for example 20-99%) of the formulation in order to provide that the tablet is not too small. However, a preferred feature of the invention is that low-melting, high dose NSAIDs, such as ibuprofen, can be formulated into smaller dosage forms. Accordingly, the NSAID will suitably form greater than 70% w/w of the granular composition (for example 70-99% by weight), preferably 70-95%, further preferably 75-85% w/w of the granular composition. The NSAID will suitably form greater than 50% by weight of the formulation, for example 60-97% by w/w, preferably 70-95% by w/w, more preferably 70-90% w/w and most preferably 75-85% w/w of the formulation.

The optional disintegrating agent has the effect of causing an NSAID tablet formulation to disintegrate under the conditions found in the gastro-intestinal tract. Examples of disintegrating agents include one or more of wheat starch, maize starch, potato starch, sodium starch glycolate, low-substituted hydroxypropyl cellulose, alginic acid, cross-linked polyvinylpyrrolidone, magnesium aluminium silicate and croscarmellose sodium. Preferred disintegrating agents are those which swell on the action of water thus causing the ingredients in the tablet to be pushed apart and out into the aqueous disintegration medium. Preferred disintegrating agents comprise one or more of croscarmellose sodium and sodium starch glycolate, especially croscarmellose sodium. The disintegrating agent may be present at an effective disintegrating amount, for example up to 25% w/w of the formulation, more preferably 1-25% w/w, further preferably 3-20% w/w and most preferably 8-17% w/w of the formulation. The disintegrating agent will suitably form 1-25% w/w of the granular composition, preferably 5-23% w/w and most preferably 8-20% by weight of the granular composition. Additionally or alternatively, the disintegrating agent may be present in the extra-granular composition.

When the granular composition includes a disintegrant, the ratio of NSAID to disintegrating agent is preferably in the range 30:1 to 1:1 parts by weight, preferably 20:1 to 2:1, more preferably 10:1 to 3:1 parts by weight.

Preferably the formulation comprises 30-99.9% w/w, more preferably 50-99.9% w/w granular composition (even more preferably 60-99% w/w, most preferably 70-99% w/w) and 0.1-70% w/w, more preferably 0.1-50% w/w extra-granular composition (even more preferably 1-40% w/w, most preferably 1-30% w/w). Further preferably the weight ratio of NSAID to said wicking agent is 2-200:1, more preferably 100-200:1.

The extra-granular composition comprises the ingredients incorporated in the compressed tablet which are not contained in the solidified melt granules. They may be mixed with the melt granules simultaneously or at sequential stages in the process to prepare the tablets. A particular advantage of the present invention is preferably that all the ingredients of the extra-granular composition are combined with the granular composition at the same time and also that there does not have to be significant processing of the ingredients in the extra-granular composition prior to combining with the granular composition. The compressed tablet comprises a uniform mixture of granular composition and extra-granular composition. The extra-granular composition is suitably distributed evenly throughout the formulation.

The wicking agent is insoluble in water and is suitably incorporated in the extra-granular composition. By "insoluble in water" it is meant that more than 10,000 ml of water is required to produce a solution using 1 gram of solid at a temperature range of 15 to 25°C. It is suitably present in the formulation to an extent of 0.1-10% w/w (preferably 0.1-5% w/w, more preferably 0.2-3% w/w and especially 0.2-1% w/w) of the formulation. Said insoluble wicking agent is selected from an inorganic material, stearic acid or insoluble salts thereof, a starch material, a cellulose material, and mixtures thereof. Preferably the inorganic material comprises talc, PTFE powder, alkali metal silicates, alkaline earth metal silicates, alkali metal carbonates and bicarbonates and alkaline earth metal carbonates. Examples include sodium carbonate, sodium bicarbonate, potassium carbonate, magnesium carbonate, calcium carbonate, talc, PTFE powder, sodium silicate, potassium silicate, magnesium silicate and calcium silicate. Preferably the stearic acid material comprises stearic acid or alkaline earth metal salts thereof, more preferably stearic acid, magnesium stearate or calcium stearate. Preferably the starch material comprises starches such as potato starch, maize starch, rice starch, tapioca starch and starch derivatives including modified starches such as pre-gelatinised starch. Most preferably, the wicking agent comprises at least one of stearic acid, magnesium stearate, calcium stearate, talc, maize starch and pre-gelatinised starch.

A suitable extra-granular composition comprises 2-100% w/w, preferably 50-100% w/w insoluble wicking agent. Preferably, the extra-granular composition comprises 85-100% w/w (more preferably 90-100% w/w, and most preferably 95-100% w/w) insoluble wicking agent. It may be combined in the extra-granular composition with 0-20% w/w preferably 0-15% w/w surfactant, more preferably 0.1-10% w/w, further preferably 0.1-5% w/w and most preferably 0.2-2% w/w.

In a particularly preferred formulation according to the present invention, the extra-granular composition consists essentially of inorganic wicking agent.

Although not necessary for the carrying out of the present invention, if desired the compressed tablet formulation may comprise additional excipients.

For example, the formulation may comprise a proportion of diluent. The diluent may be water-soluble or water-insoluble. Suitable water-soluble diluent materials include the sugar alcohols (such as xylitol, sorbitol, mannitol, erythritol), sugars (such as sucrose, fructose, lactose, dextrose), cyclodextrin, maltodextrin and salts of organic acids (eg sodium citrate and potassium citrate). Lactose, sodium citrate and potassium citrate are particularly preferred water-soluble diluents. Suitable water-insoluble diluent materials include cellulose derivatives (such as microcrystalline cellulose), starch and derivatives thereof (such as pre-gelatinised starch), dicalcium phosphate, tricalcium phosphate, calcium sulphate, and calcium carbonate. Microcrystalline cellulose and dicalcium phosphate are preferred water insoluble diluents. The diluent may preferably include a basic ingredient such as an alkali metal salt for example an alkali metal carbonate, bicarbonate or citrate. Preferably, the alkali metal salt is sodium or potassium. Further preferably, the salt is a citrate, carbonate or bicarbonate salt of sodium or potassium, more preferably sodium bicarbonate or citrate. It will be noted that the diluent may also function as a wicking agent. This dual functionality of components is within the scope of the invention.

In a formulation adapted to disperse in water prior to administration, the level of diluent may be quite high, for example up to 50% (such as 0-50% w/w, preferably 0-40% w/w) by weight of the formulation in order to achieve the desired dispersing properties. Preferably, in tablets for oral administration, the diluent does not form greater than 25% by weight of the formulation (eg 0-25% w/w), as it adds to the costs of the formulation and to production costs. Thus, to minimise costs it may be preferred that the diluent is added to the formulation in an amount of 0-20% by weight of the formulation, more preferably 0-10% w/w. If present, it may be preferably used to an extent of 0.1-25% by weight of the formulation, more preferably 0.1-20% w/w, further preferably 0.1-10% w/w and most preferably 1-5% by weight of the formulation.

The diluent may be present in the granular composition and/or the extra granular composition. To minimise the size and cost of the dosage form, it is desired to include a minimum amount of such additional excipients. If necessary for tabletting a low dose drug, the diluent material may form up to 65% w/w of the granular composition and/or the extra-granular composition (for example 0.1-60% w/w). Preferably, it is used to an extent of 0-30% w/w and more preferably 0-20% w/w of the granular composition and/or the extra-granular composition. Accordingly, if employed, the diluent may preferably be included in the extra-granular composition in the range up to 20% w/w (ie 0.1-20%), preferably 0.1-15% w/w, more preferably 0.1-10% w/w and especially 1-5% w/w. As discussed hereinabove, the diluent may be present in the granular composition, for example 0-20% w/w (such as 0.1-20% w/w) of the composition, for example 0-15% w/w and, if present, more preferably 0.1-15% w/w, especially 1-5% w/w of the formulation.

The ratio of NSAID (especially ibuprofen medicament) to alkali metal salt may be in the range 100:1 to 1:1 parts by weight, preferably 5:1 to 1:1 parts by weight.

Preferably, the alkali metal salt is incorporated in any amount up to an equimolar amount with respect to the NSAID (eg ibuprofen). Conveniently, a sub-molar amount of alkali metal salt is incorporated. Thus the alkali metal compound may form up to 100% w/w of the NSAID, preferably 50% w/w, more preferably up to 10% w/w, of the NSAID. In a preferred compressed tablet according to the present invention, the NSAID (especially an ibuprofen medicament) is in admixture with the alkali metal salt. The alkali metal salt is preferably incorporated into the extra-granular component for admixture with the granular component prior to compression into a tablet.

The formulation may also include a surfactant, in an amount appropriate to the properties of the surfactant, preferably 0.05-20% by weight of the formulation. Preferred surfactants are sodium lauryl sulphate, poloxamer, hydrogenated castor oil and derivatives thereof, polyoxyethylene surfactants (including polyoxyethylene oils, fatty acid esters, including stearates) and sorbitan esters. They may be used to an extent of 0.05-5% w/w, preferably 0.1-3% w/w, more preferably 0.2-2% w/w) of either or both the granular composition and the extra-granular composition.

Optionally a lubricant may be incorporated in the extra-granular composition for mixing with the granular composition. Conventional lubricants for ibuprofen tablets may be used for example, sodium lauryl sulphate, polyethylene glycol, hydrogenated vegetable oil and/or sodium stearyl fumarate. These may be present in an amount from 0.05-5% by weight, preferably 0.1-2% by weight of the formulation. Anti-adherents such as talc, may further be included in an amount of up to 4% by weight of the dosage form, for example 0.5-2% by weight of the dosage form, preferably as part of the extra-granular composition.

Other conventional tabletting excipients known to the person skilled in the art may be incorporated in the compressed tablet composition according to the present invention as desired, although it will be appreciated that a prime advantage of the present invention is that the number of excipients necessary to achieve a quickly disintegrating tablet with good dissolution characteristics is minimal.

A preferred granular composition comprises an NSAID (preferably ibuprofen), a disintegrant and optionally a surfactant and/or a diluent. A further preferred granular composition consists essentially of an NSAID (preferably ibuprofen), a disintegrant and a surfactant. A further preferred granular composition consists essentially of an NSAID (preferably ibuprofen), a disintegrant, a surfactant and a diluent.

The melt granules in the granular composition preferably have a mean particle size in the range 10-2000µm, more preferably 50-1000µm and most preferably 100-400µm. Valuable results are achieved when the bulk density of the melt granules is in the range 0.1-1gml⁻¹, more preferably 0.3-0.6gml⁻¹. Further preferred properties are obtained when the tapped density is in the range 0.3-0.7gml⁻¹ (more preferably 0.4-0.6 gml⁻¹ ).

A preferred compressed tablet formulation of the present invention comprises:
(a) 30-99% (preferably 60-99%) granular composition by weight of the formulation, said granular composition incorporating 0.005-3 (preferably 0.01-1) parts by weight disintegrant per part by weight of non-steroidal anti-inflammatory drug; and
(b) 0.05-70% (preferably 0.01-40%) extra-granular composition by weight of the formulation.

A preferred compressed tablet composition comprises an intimate mixture of:
(a) a granular composition comprising a solidified melt of an ibuprofen medicament incorporating a disintegrant homogeneously dispersed therein; and
(b) 0.05-30% (preferably 0.1-5%) w/w insoluble wicking agent.

In a further preferred compressed tablet composition according to the present invention, there is provided a compressed mixture of:
(a) solidified melt granules comprising 70-97% ibuprofen by weight of the granule (preferably 70-95% by weight), 3-25% croscarmellose sodium by weight of the granule (preferably 5-20% by weight) and 0-20% diluent by weight of the granule (preferably 8-16% by weight) uniformly dispersed therein, the ibuprofen being present as a continuous phase; and
(b) 0.05-5.0% w/w insoluble wicking agent.

In a further preferred composition according to the present invention there is provided, preferably as an intimate mixture,
(a) 90-99.95% granular composition by weight of the formulation, said granular composition comprising solidified melt granules of ibuprofen incorporating croscarmellose sodium and optionally a diluent uniformly dispersed therein, said ibuprofen being present in a single continuous phase and in an amount of 70-99% by weight of the composition, said croscarmellose sodium being present in an amount of 1-25% by weight of the formulation and said diluent being present in an amount of 0-20% by weight of the formulation; and
(b) 0.05%-10% extra-granular composition by weight.

An advantageous compressed tablet formulation according to the present invention comprises a uniform blend of:
(a) a granular composition comprising:
   i) 70-90% w/w ibuprofen, said ibuprofen being present as a continuous phase ;
   ii) 8-20% w/w croscarmellose sodium;
   iii) 0-20% w/w diluent; and
(b) an extra-granular composition comprising:
   iv) 0.5%-2% w/w insoluble wicking agent; and
   v) 0.1-2.5% w/w surfactant.
the sum of components (i) to (v) being greater than 99% by weight of the formulation.

Most preferably, the granular composition consists essentially (ie greater than 98% by weight of the composition) of ibuprofen, croscarmellose sodium and optionally a diluent (preferably a salt (eg an alkali metal salt) of an organic acid or microcrystalline cellulose). In a further advantageous formulation, the granular composition consists essentially of ibuprofen, croscarmellose sodium and a surfactant. Particular advantages are also achieved if the granular composition consists essentially of ibuprofen, croscarmellose sodium, a diluent (preferably microcrystalline cellulose or an alkali metal salt of an organic acid) and a surfactant (preferably sodium lauryl sulphate or a poloxamer). For example, an advantageous formulation may consist essentially of (ie greater than 98% by weight of the composition) a uniform mixture of 75-95% ibuprofen by weight of the granular composition, 5-20% disintegrant by weight of the granular composition and 0-20% diluent by weight of the granular composition, the composition comprising solidified melt granules of ibuprofen and the ibuprofen being present as a single continuous phase.

The compressed tablet formulation of the present invention may, if desired, include other compatible pharmacologically active ingredients and/or enhancing agents. Thus, for example, the dosage form may include any other ingredient commonly used in a composition useful to treat pain, inflammation and/or fever, for example caffeine or another xanthine derivative, another analgesic, for example codeine, a skeletal muscle relaxant: an antihistamine (e.g. acrivastine, astemizole, azatadine, azelastine, bromodiphenhydramine, brompheniramine, carbinoxamine, cetirizine, chlorpheniramine, cyproheptadine, dexbromopheniramine, dexchloropheniramine, diphenhydramine, ebastine, ketotifen, lodoxamide, loratidine, levocabastine, mequitazine, oxatomide, phenindamine, phenyltoloxamine, pyrilamine, setastine, tazifylline, temelastine, terfenidine, tripelennamine or triprolidine (preferably non-sedating antihistamines are employed)); a decongestant (eg pseudoephedrine, phenylpropanolamine and phenylephrine); a cough suppressant (eg caramiphen, codeine or dextrometnorpan): and/or an expectorant (eg guaifenesin, potassium citrate, potassium guaiacolsuphonate, potassium sulphate and terpin hydrate).

Such extra active ingredients and/or enhancing agents may be incorporated in the melt granules or in the extra-granular component which is combined with the melt granule prior to formulation into a compressed tablet.

Ibuprofen and its derivatives are primarily anti-inflammatory, analgesic and antipyretic agents but have also been proposed for other therapeutic uses, including the treatment of periodontal bone loss, pruritus and Alzheimer's disease. The dosage forms of the present invention are therefore indicated for use in the treatment of all therapeutic uses for which ibuprofen is effective, including rheumatoid arthritis, osteoarthritis, ankylosing spondylitis, seronegative arthropathies, periarticular disorders and soft tissue injuries. They may also be used in the treatment of postoperative pain, postpartum pain, dental pain, dysmenorrhoea, headache, migraine, rheumatic pain, muscular pain, backache, neuralgia and/or musculoskeletal pain or the pain or discomfort associated with the following: respiratory infections, colds or influenza, gout or morning stiffness.

Accordingly, in another aspect of the present invention there is provided the use of a composition according to the first aspect of the present invention for the preparation of a medicament for the treatment of pain and/or inflammation and/or fever. The formulation may be used to manufacture a medicament for the treatment of coughs, cold, influenza, headache, rheumatic pain, muscular pain or neuralgia.

Unit dosages for effective therapy are known to those skilled in the art for each NSAID. For example, they may comprise the NSAID to an extent of 5mg, 10mg, 12.5mg, 25mg, 50mg, 100mg, 150mg, 200mg, 250mg, 300mg, 350mg, 400mg, 500mg, 600mg and 800mg. Where derivatives are employed, normally the precise unit dosages are chosen to give the equivalent NSAID doses given above. For the treatments described herein the maximum daily dose of ibuprofen is generally 3200 mg. A single unit daily dose may be 100 mg. Preferred unit doses are in the range 100-400 mg, more preferably 100-300 mg and especially 200 mg ibuprofen. The maximum daily dose of flurbiprofen is generally 300 mg. A single unit dose may be 12.5 mg. Preferred unit doses are in the range 12.5-150 mg, more preferably 25-100 mg and especially 50 mg flurbiprofen. The maximum daily dose of naproxen is generally 1500 mg. A single unit daily dose may be 125 mg. Preferred unit doses are in the range 220-750 mg, more preferably 220-500 mg and especially 220-250 mg naproxen. The maximum daily dose of ketoprofen is generally 200 mg. A single unit dose may be 25 mg. Preferred unit doses are in the range 25-100 mg, more preferably 25-75 mg and especially 50 mg ketoprofen.

The tablet formulation of the present invention may be swallowed or dispersed in water prior to ingestion. Preferably the tablet composition releases the NISAID in the stomach or gastro-intestinal tract.

In a further aspect, the present invention provides the use of an insoluble wicking agent selected from an inorganic material, stearic acid or insoluble salts thereof, a starch material, a cellulose material, and a mixture thereof in an extra-granular composition combined with a granular composition in a compressed formulation, said granular composition comprising a plurality of solidified melt granules comprising a continuous phase of a low melting (e.g. melting point is in the range 30-300°C) non-steroidal anti-inflammatory drug, optionally incorporating a disintegrant and/or a diluent homogeneously dispersed therethrough, the formulation comprising 0.1-30% (preferably 0.1-10%) w/w insoluble wicking agent by weight of the formulation.

In a further aspect, the present invention provides a process to prepare a formulation according to the invention characterised by the following steps:
(a) heating a non-steroidal anti-inflammatory drug until it is molten;
(b) forming said melt into solidified melt granules;
(c) combining said solidified melt granules with an extra-granular composition comprising an insoluble wicking agent; and optionally
(d) forming into a unit dose.

In step (a) of a process according to the present invention, the drug is melted. At room temperature, the NSAIDs used in accordance with the present invention are solid. Thus, it is necessary to heat the NSAID until it is in the molten state. Under pressurised conditions, the drug may be melted at a temperature below its normal melting point. Melting may be carried out according to known methods, including for example, heating in a vessel to a temperature above the melting point of the NSAID or by extrusion in a heated extruder. The maximum temperature is determined by the stability of the molten drug and ingredients combined therewith. The drug may be heated to any convenient temperature. Generally, the higher the temperature, the more quickly the drug will melt although this must be balanced by the energy input required to heat the drug. For highest efficiency, it is generally envisaged that the NSAID will be heated to not more than 50°C, preferably 1-25°C and more preferably 5-20°C, above its melting point to keep energy costs to a minimum. A preferred heating range is 30-180°C, more preferably 35-140°C and further preferably 40-120°C.

If the NSAID is extruded, generally the work on the NSAID by the screw configuration in the extruder will also contribute to melting the NSAID, thereby reducing its external applied temperature requirement. Accordingly the extruder barrel may be heated to a temperature less than the melting point of the NSAID. For example, the normal melting point of racemic ibuprofen is 75-77°C, however under conditions of force/pressure (such as may be encountered in an extruder or similar processing device), the external applied heat necessary to melt the ibuprofen may be reduced significantly through the mechanical heat generated by the intense mixing action within the extruder. It is generally envisaged that the extruder will be heated to a temperature not less than 25°C below the melting point of the NSAID, preferably in the range from 20°C below the melting point of the drug to 50°C above the melting point of the drug, more preferably from 15°C below the melting point of the drug to 25°C above its melting point and most preferably to a temperature in the range of 10°C on each side of the melting point of the drug. Some extruders allow different zones to be heated to different temperatures in the extruder. These temperatures can be chosen as desired to ensure that the NSAID is melted, preferably fully melted, in stage (a). Preferably, the drug is heated to a temperature in the range 80-130°C, more preferably 100-120°C. When the NSAID is ibuprofen it may conveniently be heated in the range 50-130°C, more preferably 60-100°C. When heated by conventional heating means such as a water or steam bath, it is preferably heated in the range 75-90°C, more preferably 75-85°C. The ibuprofen may also be heated and subjected to conditions of force, such as by heat-extruding the ibuprofen, for example in a twin-screw extruder. The temperature of the ibuprofen in the extruder barrel is preferably in the range 66-96°C, preferably 70-82°C. Any optional excipients may be dispersed throughout the melt by conventional blending and/or stirring techniques to form a molten mixture.

In preferred embodiments where the granular composition includes a disintegrant, the disintegrant is combined with the melted NSAID, either prior to melting or after the melting process. The disintegrant is most commonly insoluble in the ibuprofen melt and a dispersion of the solid disintegrating agent within the liquid melt is produced. The dispersion is mixed so that the disintegrating agent is uniformly or homogeneously combined with the melted NSAID. A uniform mixture is thus produced.

Conveniently, heating the NSAID and blending of optional excipients (eg by stirring, agitating, kneading or extruding the NSAID) occurs at the same time. The above-mentioned process may be carried out in a number of ways. The manner in which optional excipients are combined with the drug will depend on conditions, including the drug used and the dosage thereof, the temperature to which the drug is heated, the amount of any other excipients used, the quantity of ingredients being processed and will be within the knowledge of the person skilled in the art. In one method, the NSAID is heated in a suitable vessel until molten. The disintegrating agent and any further optional components may then be added to the molten mass and thoroughly combined therewith to form a homogeneous mixture. The disintegrating agent and these optional extra excipients may also be blended into the molten NSAID simultaneously or in sequential steps.

In a further process, the non-steroidal anti-inflammatory drug may be combined in the solid state with the disintegrating agent and further optional excipients and then heated together until said non-steroidal anti-inflammatory drug is molten. It may also be desired to combine the NSAID with at least one additional excipient in the solid state (for example the disintegrating agent), then heat until said NSAID is molten and then add any further desired excipients.

In another preferred method, the NSAID and the disintegrating agent, together with any further optional ingredients to be incorporated in the melt granule, are fed into an extruder type system (preferably having first been combined by blending together). The materials are heated and mixed by kneading by the screw(s) within the extruder until the NSAID is molten and a uniform mixture of the components is produced. Preferably, the drug is melt extruded. Further preferably, the NSAID is extruded in a twin screw extruder.

In step (b), the molten mixture is formed into solidified melt granules. The melt is allowed to solidify in any manner convenient. In some cases, the melt granules may be formed whilst the drug is in the molten state (i.e. fully molten or partially molten) and then allowed to cool to form the solidified melt granules. This may be carried out, for example in a melt-extrusion process, for example the extrudate may be chopped into pellets as it passes out of the extruder and the pellets cooled in an appropriate manner, such as a cooling belt. In a further method, after heating or heat-extrusion the molten NSAID may be cooled by feeding either to a spray tower dryer or to a spray granulator in which the molten mass is sprayed into the path of a stream of cold air to form droplets and the dried solid mass collected.

Generally, it is expected that the molten mixture will be cooled to a temperature below the melting point of the drug before being formed into granules. This includes both rapid cooling and slow cooling. Preferably the molten drug is cooled rapidly. For example, the molten NSAID may be allowed to cool at room temperature or in a cooled vessel (eg water-cooled). The molten NSAID may also be poured onto cooling trays which may be static or continuously moving. Static trays may be placed in cooling cabinets. The cooled melt forms a solid and may be scraped off the belt or collected as it falls off one end of a continuously moving belt. In order to maintain a uniform mixture of the drug with the disintegrating agent and any further excipients, it may be necessary to agitate or stir the mixture during cooling. Cooling may also occur in a stream of cooled air. The molten drug may also be cooled by passing the molten mixture onto a moving cooling belt, preferably a continuously rotating cooling belt. Preferably, the belt is cooled by water. The water may be applied to the underside of the belt along its length or partially along its length as desired and according to the length of the belt, the quantity of molten drug mixture and the speed of the belt. It is especially preferred to cool the molten drug mixture at least initially by cooling means, for example until it has started to solidify. Advantageously, the belt is water-cooled along substantially the whole of its length and it is of minimum length required (e.g. 3-7m) to allow it to cool to the solid state.

The granules produced on cooling the molten drug are preferably of a suitable size for tabletting, preferably in a standard large scale tabletting machine. The melt granules in the granular composition preferably have a mean particle size in the range 10-2000µm, more preferably 50-1000µm and most preferably 100-400µm. Valuable results are achieved when the bulk density of the melt granules is in the range 0.1-1gml⁻¹, more preferably 0.3-0.6gml⁻¹. Further preferred properties are obtained when the tapped density is in the range 0.3-0.7gml⁻¹ (more preferably 0.4-0.6 gml⁻¹). Further, preferably the melt granules have a porosity of 0.5-2.0 g/ml.

The solidified melt may be formed into granules by a plurality of methods. For example, it may be pulverised or comminuted into granules. It may be milled and/or sieved. If it is cooled on a moving belt, the cooled melt may be delivered to a comminuting device such as a scraper bar and/or mill. It may also be passed through a spray device such as a spray tower or spray granulator in which the molten material is sprayed from an orifice into a stream of cooled air, allowed to congeal/solidify and then collected. If the molten NSAID is extruded, the extrudate may be cooled and then broken into conveniently sized pieces, followed by milling and or sieving. Alternatively, the extrudate may be extruded through holes and chopped into suitably sized granules for tabletting.

Preferably in stage (b) the molten drug mixture is formed into a ribbon or ribbons and is cooled on a water-cooled belt. The solidified ribbons may be milled into granules. The granular composition may be sieved to ensure that the melt granules are of the appropriate size for efficient tabletting.

In step (c), the solidified melt granules are combined with an extra-granular component comprising an insoluble wicking agent. This may be achieved by conventional mixing and blending techniques. Examples of apparatus that may be used to facilitate this process are Ribbon Blender, IBC Blender, V-Blender & Plough Blenders.

The melt granules are combined thoroughly so as to form a uniform mixture of ingredients. The acid should be distributed evenly through the formulation to maximise the taste properties.

The combined melt granules and extra granular composition may be formed into a unit dose by compressing into a tablet.

In a preferred process according to the present invention, said NSAID comprises Ibuprofen.

The invention is illustrated by the following non-limiting Examples. In the Examples, the racemic ibuprofen and racemic flurbiprofen is available from BASF Pharma, TX, USA; colloidal silicon dioxide (also known as colloidal silica) is available from Degussa, Frankfurt, DE under the trade name Aerosil 200; Croscarmellose sodium is available from the FMC Corporation, Brussels, BE under the tradename Ac-Di-Sol; and sodium starch glycolate is available from Edward Mendell, Reigate, GB under the tradename Explotab; Poloxamer is available from BASF, DE under the trade name Pluronic F68; Dicalcium phosphate is available under the trade name Emcompress; Hydrogenated Castor oil is available from BASF, DE under the trade name Cremophor RH40, Microcrystalline cellulose is available from the FMC Corporation, Brussels, BE under the trade name Avicel PH 101.

### Dissolution Measurement

The dissolution may be measured using the dissolution method described in the US Pharmacopoeia Vol. 23, page 1791, Apparatus 2 using paddles at 50 rpm and a phosphate buffer (selected at pH 7.2 and/or pH 6.0 and/or pH 5.8).

### Friability Measurement

This test for the robustness of the tablet is a standard friability test, namely the rotation of 20 tablets for a given time period at 25rpm in a friabulator (TAR 20 manufactured by ERWEKA). The following measurements may be made: -
1. The number of capped or broken tablets;
2. The % weight loss from the tablet.

### Crushing Strength (N)

The crushing strength is a measure of the hardness of a tablet. It may be measured by recording the diametrical crushing strength when the tablet was broken between the motorised jaws of a Schleuniger crushing strength tester.

### Disintegration Time (Minutes)

The disintegration time can be measured using the disintegration method described in the European Pharmacopoeia 1986, Ref V.5.1.1 (updated 1995) using tap water (pH approximately 7) as the liquid. The method provides the time by which six tablets prepared with each Example formulation disintegrates.

### Example 1

| | **(% w/w)** |
|---|---|
| Granular composition: | |
| Ibuprofen | 87.4 |
| Croscarmellose Sodium | 7.7 |
| Extra-granular composition: | |
| Talc | 4.0 |
| Stearic Acid | 0.9 |

### Example 1(a): Preparation of Granular Component

In the illustrative process, the ibuprofen may be melted by heating to approximately 75°C in a stainless steel vessel until fully molten. The disintegrating agent (croscarmellose sodium) may then be added to the molten ibuprofen and mixed for 5-10 minutes until uniformly dispersed. The molten mixture may be poured onto a stainless steel tray and cooled over a period of up to 60 minutes, ensuring that the suspension is maintained. The mass thus formed may be milled by passing through a cone mill having a screen with a round hole size of 1 mm. The resulting granules are collected.

### Example 1(b): Preparation of Tablets

The ingredients of the extra-granular composition may be blended simultaneously with the granular composition for approximately 15 minutes in a blender. The blended material may be fed to a tabletting machine and compressed into tablets containing 200mg ibuprofen.

### Examples 2-4

| | **Ex2** **(% w/w)** | **Ex3** **(% w/w)** | **Ex4** **(% w/w)** |
|---|---|---|---|
| Granular composition: | | | |
| Ibuprofen | 91.3 | 89.7 | 85.8 |
| Croscarmellose Sodium | 7.3 | 9.0 | 12.9 |
| Extra-granular composition: | | | |
| Calcium Stearate | 0.5 | 0.4 | 0.4 |
| Stearic Acid | 0.9 | 0.9 | 0.9 |

Examples 2-4 may be prepared in the same manner as described in Example 1 to produce tablets containing 200 mg ibuprofen.

### Examples 5-8

| | **Ex5** **(% w/w)** | **Ex6** **(% w/w)** | **Ex7** **(% w/w)** | **Ex8** **(% w/w)** |
|---|---|---|---|---|
| Granular composition: | | | | |
| Ibuprofen | 93.9 | 91.3 | 89.7 | 85.8 |
| Sodium Starch Glycolate | 4.7 | 7.3 | 9.0 | 12.9 |
| Extra-granular composition: | | | | |
| Calcium Stearate | 0.5 | 0.5 | 0.4 | 0.4 |
| Stearic Acid | 0.9 | 0.9 | 0.9 | 0.9 |

Examples 5-8 may be prepared in the same manner as described in Example 1 except that sodium starch glycolate is used as the disintegrating agent. Tablets containing 200 mg ibuprofen are prepared.

### Examples 9-12

| | **Ex9** **(% w/w)** | **Ex10** **(% w/w)** | **Ex11** **(% w/w)** | **Ex12** **(% w/w)** |
|---|---|---|---|---|
| Granular composition: | | | | |
| Ibuprofen | 66.2 | 73.9 | 63.3 | 86.6 |
| Croscarmellose Sodium | 5.3 | 5.9 | 5.1 | 6.9 |
| Extra-granular composition: | | | | |
| D ibasic Calcium Phosphate Dihydrate | 1.0 | 1.1 | 0.6 | 1.3 |
| Stearic Acid | 0.7 | 0.7 | 0.9 | 0.9 |
| Sodium carbonate | - | 18.4 | - | - |
| Sodium Bicarbonate | 26.8 | - | - | - |
| Sodium citrate | - | - | 30.1 | 4.3 |

Examples 9-12 are prepared in the same manner as described in Example 1, except that a basic excipient (sodium citrate/sodium carbonate/sodium bicarbonate) is included in the extra-granular component for combination with the granular component. Tablets or component containing 200 mg ibuprofen are prepared.

### Examples 13-17

| | **Ex 13** **(% w/w)** | **Ex 14** **(% w/w)** | **Ex 15** **(% w/w)** | **Ex 16** **(% w/w)** | **Ex 17** **(% w/w)** |
|---|---|---|---|---|---|
| Granular composition: | | | | | |
| Ibuprofen | 85.7 | 85.4 | 85.1 | 84.7 | 84.4 |
| Croscarmellose Sodium | 12.9 | 12.8 | 12.8 | 12.7 | 12.7 |
| Extra-granular composition: | | | | | |
| Calcium Carbonate | 0.5 | 0.9 | 1.2 | 1.7 | 2.1 |
| Stearic Acid | 0.9 | 0.9 | 0.9 | 0.9 | 0.8 |

Examples 13-17 are prepared in the same manner as described in Example 1 to produce tablets containing 200 mg ibuprofen.

### Examples 18-21

| | **Ex 18** **(%w/w)** | **Ex 19** **(%w/w)** | **Ex 20** **(%w/w)** | **Ex 21** **(%w/w)** |
|---|---|---|---|---|
| Granular composition: | | | | |
| Ibuprofen | 79.0 | 84.1 | 84.0 | 85.7 |
| Croscarmellose Sodium | 10.3 | 11.0 | 11.0 | 11.3 |
| Poloxamer | 7.9 | 2.1 | - | - |
| Sodium Lauryl Sulphate | - | - | 2.2 | 0.2 |
| Extra-granular composition: | | | | |
| Talc | 2.0 | 2.0 | 2.0 | 2.0 |
| Stearic Acid | 0.8 | 0.8 | 0.8 | 0.8 |

Examples 18-21 are prepared in the same manner as described in Example 1, except that a surfactant (Poloxamer/sodium lauryl sulphate) is dispersed within the molten ibuprofen after the croscarmellose sodium is dispersed uniformly within the molten ibuprofen. Tablets containing 200mg ibuprofen are prepared.

### Examples 22-26

| | **Ex 22** **(% w/w)** |
|---|---|
| Granular composition: | |
| Ibuprofen | 82.8 |
| Croscarmellose sodium | 12.3 |
| Extra-granular composition: | |
| Microcrystalline cellulose | - |
| Lactose | - |
| Dicalcium phosphate | 4.1 |
| Colloidal silicon dioxide | - |
| Stearic acid | 0.8 |

Example 22 is prepared in the same manner as described in Example 1, except that a diluent (microcrystalline cellulose/lactose/dicalcium phosphate) is included in the extra-granular component. Tablets containing 200 mg ibuprofen are prepared.

### Examples 23-24

| | **Ex 23** **(% w/w)** | **Ex 24** **(% w/w)** |
|---|---|---|
| Granular composition: | | |
| Ibuprofen | 79.0 | 70.6 |
| Croscarmellose sodium | 11.9 | 10.6 |
| Microcrystalline cellulose | 7.9 | - |
| Dicalcium phosphate | - | 17.6 |
| Extra-granular composition: | | |
| Calcium Stearate | 0.4 | 0.4 |
| Stearic acid | 0.8 | 0.8 |

Examples 23 and 24 are prepared in the same manner as described in Example 1, except that a diluent (microcrystalline cellulose/dicalcium phosphate) is dispersed within the molten ibuprofen after the croscarmellose sodium had been dispersed uniformly within the molten ibuprofen. Tablets containing 200 mg ibuprofen are prepared.

### Example 25

| | **Example 25 (%w/w)** |
|---|---|
| Granular composition: | |
| Ibuprofen | 84 |
| Sodium starch glycolate | 8 |
| Extra-granular composition: | |
| Microcrystalline cellulose | - |
| Croscarmellose sodium | 5 |
| Sodium lauryl sulphate | 1 |
| Dibasic calcium phosphate | 1 |
| Poloxamer | 1 |

Example 25 was prepared in the same manner as described in Example 1, using sodium starch glycolate as the disintegrating agent.

The dissolution data, tablet hardness, friability and tablet disintegration results are given below.

| **Dissolution results at pH 7.2** | |
|---|---|
| **Time (min)** | **Ex 25** |
| 0 | 0.0 |
| 10 | 73.7 |
| 20 | 90.2 |
| 30 | 95.9 |
| 45 | 98.2 |
| 60 | 98.6 |

| **Tablet Hardness, Friability and Disintegration Time** | |
|---|---|
| | **Ex 25** |
| **Tablet hardness (Kp)** | 2.9 |
| **Friability (as a fraction of tablet number)** | 1/10 |
| **Disintegration time (seconds)** | 45 |

### Examples 26-27

| | **Ex 26 (%w/w)** | **Ex 27 (%w/w)** |
|---|---|---|
| Granular composition: | | |
| Ibuprofen | 85.8 | 81.2 |
| Croscarmellose sodium | 12.9 | 12.9 |

| Extra-granular composition: | | |
|---|---|---|
| Stearic acid | 0.9 | 0.9 |
| Talc | - | - |
| Calcium stearate | 0.4 | 5 |

Examples 26-27 are prepared in the same manner as described in Example 1, except that calcium stearate is used instead of talc in the extra-granular composition. Tablets containing 200 mg ibuprofen are prepared.

### Examples 28-30

| | **Ex 28 (%wlw)** | **Ex 29 (%w/w)** | **Ex 30 (%w/w)** |
|---|---|---|---|
| Granular composition: | | | |
| Ibuprofen | 86 | 74 | 79.9 |
| Croscarmellose sodium | - | 5 | - |
| Sodium starch glycolate | 12.9 | 10 | 7.7 |
| Microcrystalline cellulose | - | - | 7.7 |
| Extra-granular compositions: | | | |
| Sodium lauryl sulphate | - | 1 | 1 |
| Magnesium silicate | - | - | 0.7 |
| Stearic acid | 0.9 | - | 1 |
| Talc | 0.2 | 10 | - |
| Dibasic calcium phosphate | - | - | 1 |
| Poloxamer | - | - | 1 |

Examples 28-30 are prepared in the same manner as described in Example 1, except that different disintegrants are used in the granular composition and different components are used in the extra-granular composition. Tablets containing 200 mg ibuprofen are prepared.

### Examples 31-33

| | **Ex 31 (%w/w)** | **Ex 32 (%w/w)** | **Ex 33 (%w/w)** |
|---|---|---|---|
| Granular composition: | | | |
| Flurbiprofen | 45.5 | 71 | 71 |
| Croscarmellose sodium | 7.2 | 5 | 13 |
| Sodium starch glycolate | - | 13 | 5 |
| Microcrystalline cellulose | 45.5 | 9 | 9 |
| Extra-granular composition: | | | |
| Magnesium silicate | 0.9 | - | - |
| Stearic acid | 0.9 | 1 | 1 |
| Talc | - | 1 | 1 |

Examples 31-33 are prepared in the same manner as described in Example 1, except that flurbiprofen is used instead of ibuprofen and different excipients are used in the granular and extra-granular compositions. The flurbiprofen was heated at 120°C until fully molten. Tablets containing 50 mg flurbiprofen are prepared.

### Examples 33-35

| | **Ex 34 (%w/w)** | **Ex 35 (%w/w)** |
|---|---|---|
| Granular compositions: | | |
| Ketoprofen | 31.3 | 50 |
| Croscarmellose sodium | 5 | 10 |
| Sodium starch glycolate | - | 5 |
| Microcrystalline cellulose | 62.5 | - |
| Extra-granular compositions: | | |
| Magnesium silicate | - | 1 |
| Stearic acid | 0.6 | - |
| Talc | 0.6 | - |
| Magnesium stearate | - | 1 |
| Lactose | - | 33 |

Examples 34-35 are prepared in the same manner as described in Example 1, except that ketoprofen is used instead of ibuprofen and different excipients are used in the granular and extra-granular compositions. The ketoprofen was heated at 100°C until fully molten. Tablets containing 50 mg ketoprofen are prepared.

### Examples 36-37

| | **Ex 36 (%w/w)** | **Ex 37 (%w/w)** |
|---|---|---|
| Granular compositions: | | |
| Naproxen | 66.2 | 66.2 |
| Croscarmellose sodium | 5.3 | 5.3 |
| Sodium starch glycolate | - | 10 |
| Microcrystalline cellulose | - | 11.8 |
| Extra-granular compositions: | | |
| Maize starch | 26.8 | - |
| Stearic acid | 0.7 | 0.7 |
| Dibasic calcium phosphate | 1 | 1 |
| Sodium bicarbonate | - | 5 |

Examples 36-37 are prepared in the same manner as described in Example 1, except that naproxen is used instead of ibuprofen and different excipients are used in the granular and extra-granular compositions. The naproxen was heated at 170°C until fully molten. Tablets containing 250 mg naproxen are prepared.

### Examples 38-40

| | **Ex 38 (%w/w)** | **Ex 39 (%w/w)** | **Ex 40 (%w/w)** |
|---|---|---|---|
| Granular compositions: | | | |
| Ibuprofen | 50 | 58 | 50 |
| Croscarmellose sodium | 10 | 10 | 10 |
| Sodium starch glycolate | 5 | 5 | 5 |
| Microcrystalline cellulose | 7 | - | 13 |
| Extra-granular compositions: | | | |
| Maize starch | - | - | 15 |
| Hydroxypropyl starch | - | 25 | - |
| Magnesium stearate | 1 | 1 | 1 |
| Dibasic calcium phosphate | 1 | 1 | 1 |
| Lactose | 25 | - | 5 |
| Poloxamer | 1 | - | - |

Examples 38-40 are prepared in the same manner as described in Example 1, except that different excipients are used in the granular and extra-granular compositions. Tablets containing 200 mg ibuprofen are prepared.

### Examples 41-43

| | **Ex 41 (%w/w)** | **Ex 42 (%w/w)** | **Ex 43 (%w/w)** |
|---|---|---|---|
| Granular compositions: | | | |
| Ibuprofen | 91.3 | 81.2 | 70.6 |
| Croscarmellose sodium | 7.3 | - | 11.4 |
| Sodium starch glycolate | - | 12.9 | - |
| Extra-granular compositions: | | | |
| Hydroxypropyl starch | - | 5 | - |
| Stearic acid | 0.9 | 0.9 | - |
| Calcium stearate | 0.5 | - | 0.4 |
| Dibasic calcium phosphate | - | - | 17.6 |

Examples 41-43 are prepared in the same manner as described in Example 1, except that different excipients are used in the granular and extra-granular compositions. Tablets containing 200 mg ibuprofen are prepared.

### Examples 44-46

| | **Ex 44 (%w/w)** | **Ex 45 (%w/w)** | **Ex 46 (%w/w)** |
|---|---|---|---|
| Granular compositions: | | | |
| Ibuprofen | 85.7 | 83.4 | 66.2 |
| Croscarmellose sodium | 11.3 | 12.7 | 5.3 |
| Extra-granular compositions: | | | |
| Sodium lauryl sulphate | 0.2 | - | - |
| Pregelled starch | - | 1 | - |
| Stearic acid | - | 0.8 | 0.7 |
| Talc | 2 | - | - |
| Magnesium stearate | 0.8 | - | - |
| Dibasic calcium phosphate | - | - | 1 |
| Calcium carbonate | - | 2.1 | - |
| Sodium bicarbonate | - | - | 26.8 |

Examples 44-46 are prepared in the same manner as described in Example 1, except that different excipients are used in the granular and extra-granular compositions. Tablets containing 200 mg ibuprofen are prepared.

### Example 47

The example formulations 1 to 46 may be prepared in the same manner as Example 1, except that the granular components (i.e. NSAID and excipients) are first mixed together in the solid state and then heated until the NSAID is fully molten.

### Example 48

The example formulations 1 to 47 may be prepared in the same manner as Example 1, except that the disintegrant component is added as part of the extra-granular composition instead of the granular composition.

## Claims

1. A formulation comprising:
(a) a granular composition comprising a plurality of solidified melt granules comprising a continuous phase of a non-steroidal anti-inflammatory drug (NSAID) wherein the NSAID is selected from racemic naproxen, racemic flurbiprofen, racemic ibuprofen and racemic ketoprofen, and the plurality of solidified melt granules are obtainable by fully melting the NSAID, and
(b) an extra-granular composition comprising a wicking agent which is insoluble in water and comprises at least one of an inorganic material, stearic acid or an insoluble salt thereof, a starch material, a cellulose material or a mixture thereof, provided that the formulation does not contain silicon dioxide.

2. A formulation according to Claim 1 in the form of a compressed tablet.

3. A formulation according to Claims 1 or 2, wherein the NSAID has a melting point of from 30 to 300 °C.

4. A formulation according to any preceding claim further comprising a disintegrant.

5. A formulation according to Claim 4, wherein the disintegrant is uniformly dispersed in the granular composition.

6. A formulation according to Claim 4, wherein the disintegrant is uniformly dispersed in the extra-granular composition.

7. A formulation according to any preceding claim comprising:
(a) 30-99.9 wt % granular composition; and
(b) 0.1-70 wt % extra-granular composition.

8. A formulation according to Claim 4 comprising:
(a) 50-99.9 wt % granular composition; and
(b) 0.1-50 wt % extra-granular composition.

9. A formulation according to any one of the preceding claims, wherein the wicking agent is present in an amount of from 0.1 to 30% by weight.

10. A formulation according to anyone of the preceding claims, wherein said extra-granular composition comprises 2-100% wicking agent.

11. A formulation according to any one of the preceding claims comprising said NSAID in a weight ratio to said wicking agent of 2.1 to 200:1.

12. A formulation according to any one of the preceding claims, wherein the NSAID comprises racemic ibuprofen.

13. A formulation according to any one of the preceding claims, wherein said inorganic material comprises at least one of talc, PTFE powder, alkaline earth metal silicates, alkali metal carbonates and alkaline earth metal carbonates.

14. A formulation according to any one of the preceding claims, wherein the inorganic material comprises at least one of sodium carbonate, sodium bicarbonate, potassium carbonate, potassium bicarbonate, magnesium carbonate, calcium carbonate, talc, PTFE powder, magnesium silicate and calcium silicate.

15. A formulation according to any one of the preceding claims, wherein said stearic acid or insoluble salt thereof comprises at least one of stearic acid, magnesium stearate and calcium stearate.

16. A formulation according to any one of the preceding claims, wherein the starch material comprises at least one of potato starch, maize starch, pre-gelatinised starch, rice starch and tapioca starch.

17. A formulation according to any one of the preceding claims, wherein the granular composition comprises 1-25% disintegrant.

18. A formulation according to any one of the preceding claims, wherein the disintegrant comprises at least one of croscarmellose sodium and sodium starch glycolate.

19. A formulation according to any one of the preceding claims further comprising a surfactant.

20. A formulation according to Claim 19, wherein the wicking agent comprises at least one of stearic acid, magnesium stearate, calcium stearate, talc, maize starch and pre-gelatinised starch.

21. A formulation according to any one of the preceding claims, wherein the extra-granular composition comprises:
(a) 2-100% w/w said wicking agent; and
(b) 0-20% w/w surfactant.

22. A formulation according to any one of the preceding claims, wherein the extra-granular composition consists essentially of the wicking agent.

23. A formulation according to any one of the preceding claims comprising:
(a) 50-90% w/w ibuprofen;
(b) 5-20% w/w croscarmellose sodium;
(c) 0.1-30% w/w said wicking agent; and
(d) 0-20% w/w surfactant.

24. A formulation according to Claims 21 or 23 wherein the surfactant comprises sodium lauryl sulphate or poloxamer or a mixture thereof.

25. A formulation according to any one of the preceding claims further comprising a diluent in an amount of 0.1 - 65% w/w.

26. A process for the preparation of a formulation according to any one of the preceding claims comprises:
(a) heating a NSAID selected from racemic naproxen, racemic flurbiprofen, racemic ibuprofen and racemic ketoprofen, until the NSAID is fully molten;
(b) forming the melt into solidified melt granules; and
(c) combining the solidified melt granules with an extra-granular composition comprising the wicking agent.

27. A process according to Claim 26 wherein the granular composition includes a disintegrant uniformly dispersed therein, and step (a) comprises:
(i) combining the disintegrant with the NSAID in the solid state and heating the mixture until the NSAID is fully molten; or
(ii) heating the NSAID until it is fully molten and adding the disintegrant thereto with mixing to form a mixture thereof.

28. A process according to Claim 27, in which the NSAID and the disintegrant are uniformly mixed in the solid state prior to heating.

29. A process according to Claim 27, comprising:
(a) heating the NSAID until it is fully molten and then combining the molten NSAID with the disintegrant to form a mixture thereof;
(b) forming said mixture into solidified melt granules with cooling;
(c) combining said solidified melt granules with an extra-granular composition comprising the wicking agent.

30. A process according to any one of Claims 26 to 29, wherein the mixture is cooled to the solid state before being formed into granules.

31. A process according to any one of Claims 26 to 30, wherein the ingredients of the extra-granular composition are added sequentially to the granular composition with stirring.

32. A process according to any one of Claims 26 to 31, wherein the NSAID comprises racemic ibuprofen.

33. A formulation according to any one of Claims 1 to 25 in the form of a unit dose.

34. The use of a formulation according to any one of Claims 1 to 25 in the manufacture of a medicament for the treatment of pain and/or inflammation and/or fever.

35. Use according to claim 34 in the manufacture of a medicament for the treatment of coughs, colds, influenza, headache, rheumatic pain, muscular pain or neuralgia.

## Patentansprüche

1. Formulierung, umfassend:
(a) eine granuläre Zusammensetzung, umfassend eine Vielzahl von Granulatkörnern aus erstarrter Schmelze, umfassend eine kontinuierliche Phase eines nichtsteroidalen entzündungshemmenden Arzneimittels (NSAID), wobei das NSAID aus racemischem Naproxen, racemischem Flurbiprofen, racemischem Ibuprofen und racemischem Ketoprofen ausgewählt ist und die Vielzahl von Granulatkörnern aus erstarrter Schmelze durch vollständiges Schmelzen des NSAID gewinnbar ist, und
(b) eine extragranuläre Zusammensetzung, umfassend ein Saugmittel, das in Wasser unlöslich ist und wenigstens einen der folgenden Stoffe umfasst: ein anorganisches Material, Stearinsäure oder ein unlösliches Salz derselben, ein Stärkematerial, ein Cellulosematerial oder eine Mischung daraus, vorausgesetzt, dass die Formulierung kein Siliciumdioxid enthält.

2. Formulierung gemäß Anspruch 1 in Form einer gepressten Tablette.

3. Formulierung gemäß Anspruch 1 oder 2, wobei das NSAID einen Schmelzpunkt von 30 bis 300 °C hat.

4. Formulierung gemäß einem der vorhergehenden Ansprüche, weiterhin ein Sprengmittel umfassend.

5. Formulierung gemäß Anspruch 4, wobei das Sprengmittel gleichmäßig in der granulären Zusammensetzung verteilt ist.

6. Formulierung gemäß Anspruch 4, wobei das Sprengmittel gleichmäßig in der extragranulären Zusammensetzung verteilt ist.

7. Formulierung gemäß einem der vorhergehenden Ansprüche, umfassend:
(a) 30 - 99,9 Gew.-% granuläre Zusammensetzung und
(b) 0,1 - 70 Gew.-% extragranuläre Zusammensetzung.

8. Formulierung gemäß Anspruch 4, umfassend:
(a) 50 - 99,9 Gew.-% granuläre Zusammensetzung und
(b) 0,1 - 50 Gew.-% extragranuläre Zusammensetzung.

9. Formulierung gemäß einem der vorhergehenden Ansprüche, wobei das Saugmittel in einer Menge von 0,1 bis 30 % nach Gewicht vorhanden ist.

10. Formulierung gemäß einem der vorhergehenden Ansprüche, wobei die extragranuläre Zusammensetzung 2 - 100 % Saugmittel umfasst.

11. Formulierung gemäß einem der vorhergehenden Ansprüche, die das NSAID bezogen auf das Saugmittel in einem Gewichtsverhältnis von 2,1 bis 200:1 umfasst.

12. Formulierung gemäß einem der vorhergehenden Ansprüche, wobei das NSAID racemisches Ibuprofen umfasst.

13. Formulierung gemäß einem der vorhergehenden Ansprüche, wobei das anorganische Material wenigstens einen der Stoffe Talcum, PTFE-Pulver, Erdalkalimetallsilikate, Alkalimetallcarbonate und Erdalkalimetallcarbonate umfasst.

14. Formulierung gemäß einem der vorhergehenden Ansprüche, wobei das anorganische Material wenigstens einen der Stoffe Natriumcarbonat, Natriumbicarbonat, Kaliumcarbonat, Kaliumbicarbonat, Magnesiumcarbonat, Calciumcarbonat, Talcum, PTFE-Pulver, Magnesiumsilicat und Calciumsilicat umfasst.

15. Formulierung gemäß einem der vorhergehenden Ansprüche, wobei die Stearinsäure oder das unlösliche Salz derselben wenigstens einen der Stoffe Stearinsäure, Magnesiumstearat und Calciumstearat umfasst.

16. Formulierung gemäß einem der vorhergehenden Ansprüche, wobei das Stärkematerial wenigstens einen der Stoffe Kartoffelstärke, Maisstärke, vorgelatinierte Stärke, Reisstärke und Tapiokastärke umfasst.

17. Formulierung gemäß einem der vorhergehenden Ansprüche, wobei die granuläre Zusammensetzung 1 - 25 % Sprengmittel umfasst.

18. Formulierung gemäß einem der vorhergehenden Ansprüche, wobei das Sprengmittel wenigstens einen der Stoffe Croscarmellose-Natrium und Natriumstärkeglycolat umfasst.

19. Formulierung gemäß einem der vorhergehenden Ansprüche, weiterhin ein oberflächenaktives Mittel umfassend.

20. Formulierung gemäß Anspruch 19, wobei das Saugmittel wenigstens einen der Stoffe Stearinsäure, Magnesiumstearat, Calciumstearat, Talcum, Maisstärke und vorgelatinierte Stärke umfasst.

21. Formulierung gemäß einem der vorhergehenden Ansprüche, wobei die extragranuläre Zusammensetzung umfasst:
(a) 2 - 100 % (w/w) des Saugmittels und
(b) 0 - 20 % (w/w) oberflächenaktives Mittel.

22. Formulierung gemäß einem der vorhergehenden Ansprüche, wobei die extragranuläre Zusammensetzung im Wesentlichen aus dem Saugmittel besteht.

23. Formulierung gemäß einem der vorhergehenden Ansprüche, umfassend:
(a) 50 - 90 % (w/w) Ibuprofen;
(b) 5 - 20 % (w/w) Croscarmellose-Natrium;
(c) 0,1 - 30 % (w/w) des Saugmittels und
(d) 0 - 20 % (w/w) oberflächenaktives Mittel.

24. Formulierung gemäß Anspruch 21 oder 23, wobei das oberflächenaktive Mittel Natriumlaurylsulfat oder Poloxamer oder eine Mischung daraus umfasst.

25. Formulierung gemäß einem der vorhergehenden Ansprüche, weiterhin ein Verdünnungsmittel in einer Menge von 0,1 - 65 % (w/w) umfassend.

26. Verfahren zur Herstellung einer Formulierung gemäß einem der vorhergehenden Ansprüche, umfassend:
(a) Erwärmen eines NSAID, das aus racemischem Naproxen, racemischem Flurbiprofen, racemischem Ibuprofen und racemischem Ketoprofen ausgewählt ist, bis das NSAID vollständig geschmolzen ist;
(b) Ausbilden der Schmelze zu Granulatkörnern aus erstarrter Schmelze und
(c) Kombinieren der Granulatkörner aus erstarrter Schmelze mit einer extragranulären Zusammensetzung, die das Saugmittel umfasst.

27. Verfahren gemäß Anspruch 26, wobei die granuläre Zusammensetzung ein gleichmäßig darin verteiltes Sprengmittel enthält und Schritt (a) umfasst:
(i) Kombinieren des Sprengmittels mit dem NSAID im festen Zustand und Erwärmen der Mischung, bis das NSAID vollständig geschmolzen ist; oder
(ii) Erwärmen des NSAID, bis es vollständig geschmolzen ist, und Zugabe des Sprengmittels dazu unter Mischen, um eine Mischung daraus zu bilden.

28. Verfahren gemäß Anspruch 27, wobei das NSAID und das Sprengmittel vor dem Erwärmen im festen Zustand gleichmäßig gemischt werden.

29. Verfahren gemäß Anspruch 27, umfassend:
(a) Erwärmen des NSAID, bis es vollständig geschmolzen ist, und nachfolgendes Kombinieren des geschmolzenen NSAID mit dem Sprengmittel, um eine Mischung daraus zu bilden;
(b) Ausbilden der Mischung zu Granulatkörnern aus erstarrter Schmelze unter Abkühlung;
(c) Kombinieren der Granulatkörner aus erstarrter Schmelze mit einer extragranulären Zusammensetzung, die das Saugmittel umfasst.

30. Verfahren gemäß einem der Ansprüche 26 bis 29, wobei die Mischung in den festen Zustand abgekühlt wird, bevor sie zu Granulatkörnern ausgebildet wird.

31. Verfahren gemäß einem der Ansprüche 26 bis 30, wobei die Inhaltsstoffe der extragranulären Zusammensetzung sequentiell unter Rühren zu der granulären Zusammensetzung zugegeben werden.

32. Verfahren gemäß einem der Ansprüche 26 bis 31, wobei das NSAID racemisches Ibuprofen umfasst.

33. Formulierung gemäß einem der Ansprüche 1 bis 25 in Form einer Dosierungseinheit.

34. Verwendung einer Formulierung gemäß einem der Ansprüche 1 bis 25 bei der Herstellung eines Medikaments zur Behandlung von Schmerzen und/oder Entzündung und/oder Fieber.

35. Verwendung gemäß Anspruch 34 bei der Herstellung eines Medikaments zur Behandlung von Husten, Erkältungen, Grippe, Kopfschmerzen, rheumatischen Schmerzen, Muskelschmerzen oder Neuralgie.

## Revendications

1. Une formulation comprenant :
(a) une composition granulaire comprenant une pluralité de granulés fondus solidifiés comprenant une phase continue d'un médicament anti inflammatoire non stéodidien (AINS) où le AINS est sélectionné parmi le naproxène racémique, le flurbiprofène racémique, l'ibuprofène racémique et le kétoprofène racémique et la pluralité de granulés fondus solidifiés peuvent être obtenus en fondant complètement AINS, et
(b) une composition extra granulaire comprenant un agent absorbant qui n'est pas soluble dans l'eau et comprend au moins un parmi un matériau inorganique, un acide stéarique ou un sel insoluble de ceux-ci, un matériau d'amidon, un matériau de cellulose ou un mélange de ceux-ci, à condition que la formulation ne contient pas de silice.

2. Une formulation selon la revendication 1 dans la forme d'un comprimé.

3. Une formulation selon les revendications 1 ou 2, où AINS a un point de fusion de 30 à 300°C.

4. Une formulation selon l'une des revendications précédentes comprenant de plus un délitant.

5. Une formulation selon la revendication 4 où le délitant est dispersé uniformément dans la composition granulaire.

6. Une formulation selon la revendication 4, où le délitant est uniformément dispersé dans la composition extra granulaire.

7. Une formulation selon l'une des revendications précédentes comprenant :
(a) 30-99,9% en poids de composition granulaire ; et
(b) 0,1-70% en poids de composition extra granulaire.

8. Une formulation selon la revendication 4 comprenant :
(a) 50-99,9% en poids de composition granulaire ; et
(b) 0,1-50% en poids de composition extra granulaire.

9. Une formulation selon l'une des revendications précédentes, où l'agent absorbant est présent dans un montant de 0,1 à 30% par poids.

10. Une formulation selon l'une des revendications précédentes, où ladite composition extra granulaire comprend 2-100% d'agent absorbant.

11. Une formulation selon l'une des revendications précédentes comprenant ledit AINS dans un ratio de poids par rapport audit agent absorbant de 2:1 à 200:1.

12. Une formulation selon l'une des revendications précédentes, où le AINS comprend de l'ibuprofène racémique.

13. Une formulation selon l'une des revendications précédentes, où ledit matériau inorganique comprend au moins un parmi le talc, la poudre polytétrafluoroéthylène, les silicates de métal alcalino terreux, les carbonates de métal alcalin, les carbonates de métal alcalino terreux.

14. Une formulation selon l'une des revendications précédentes, où le matériau inorganique comprend au moins un parmi le carbonate de sodium, le bicarbonate de sodium, le carbonate de potassium, le bicarbonate de potassium, le carbonate de magnésium, le carbonate de calcium, le talc, la poudre polytétrafluoroéthylène, le silicate de magnésium, et le silicate de calcium.

15. Une formulation selon l'une des revendications précédentes, où l'acide stéarique ou le sel insoluble de celui-ci comprend au moins un parmi l'acide stéarique, le stéarate de magnésium et le stéarate de calcium.

16. Une formulation selon l'une des revendications précédentes, où le matériau d'amidon comprend au moins un parmi l'amidon de pommes de terre, l'amidon de mais, l'amidon prégélatinisé, l'amidon de riz et le tapioca.

17. Une formulation selon l'une des revendications précédentes, où la composition granulaire comprend 1-25% de délitant.

18. Une formulation selon l'une des revendications précédentes, où le délitant comprend au moins un parmi le croscarmellose sodium et le glycolate d'amidon sodique.

19. Une formulation selon l'une des revendications précédentes comprenant de plus un surfactant.

20. Une formulation selon la revendication 19 où l'agent absorbant comprend au moins un parmi un acide stéarique, le stéarate de magnésium, le stéarate de calcium, le talc, l'amidon de mais, et l'amidon prégélatinisé.

21. Une formulation selon l'une des revendications précédentes où la composition extra granulaire comprend :
(a) 2-100% p/p dudit agent absorbant, et
(b) 0-20% p/p surfactant.

22. Une formulation selon l'une des revendications précédentes où la composition extra granulaire consiste essentiellement en l'agent absorbant.

23. Une formulation selon l'une des revendications précédentes comprenant :
(a) 50-90% p/p ibuprofène
(b) 5-20% p/p croscarmellose sodium
(c) 0.1-30% p/p dudit agent absorbant et
(d) 0-20% p/p surfactant.

24. Une formulation selon les revendications 21 ou 23 où le surfactant comprend du laurylsulfate de sodium ou du poloxamère ou un mélange de cela.

25. Une formulation selon l'une des revendications précédentes comprenant de plus un diluant dans un montant de 0,1 à 65% p/p.

26. Un processus pour la préparation d'une formulation selon l'une des revendications précédentes comprend :
(a) le chauffage de AINS sélectionné parmi le naproxène racémique, le flurbiprofène racémique, l'ibuprofène racémique et le kétoprofène racémique jusqu'à ce que AINS soit complètement fondu ;
(b) la formation de la fonte en granulés fondus solidifiés ; et
(c) la combinaison des granulés fondus solidifiés avec une composition extra granulaire comprenant l'agent absorbant.

27. Un processus selon la revendication 26 où la composition granulaire inclut un délitant uniformément dispersé là-dedans et l'étape (a) comprend :
(i) combiner le délitant avec AINS dans l'état solide et chauffer le mélange jusqu'à ce que AINS soit complètement fondu ; ou
(ii) chauffer l'AINS jusqu'à ce qu'il ait fondu complètement et y ajouter le délitant sous mélange pour former un mélange de cela.

28. Un processus selon la revendication 27, dans lequel le AINS et le délitant sont uniformément mélangés dans l'état solide avant le chauffage.

29. Un processus selon la revendication 27, comprenant :
(a) le chauffage de AINS jusqu'à ce que AINS soit complètement fondu et alors la combinaison de AINS fondu avec le délitant pour former un mélange de cela ;
(b) la formation du mélange en granulés fondus solidifiés sous refroidissement ;
(c) la combinaison des granulés fondus solidifiés avec une composition extra granulaire comprenant l'agent absorbant.

30. Un processus selon l'une des revendications 26 à 29 où la mélange est refroidi jusqu'à l'état solide avant d'être formé en granulés.

31. Un processus selon l'une des revendications 26 à 30, où les ingrédients de la composition extra granulaire sont ajoutés séquentiellement à la composition granulaire avec agitation.

32. Un processus selon l'une des revendications 26 à 31 où AINS comprend l'ibuprofène racémique.

33. Une formulation selon l'une des revendications 1 à 25 dans la forme d'une unidose.

34. Utilisation de la formulation selon l'une des revendications 1 à 25 dans la fabrication d'un médicament pour le traitement de douleur et/ou inflammation et/ou fièvre.

35. Utilisation selon la revendication 34 dans la fabrication d'un médicament pour le traitement de toux, rhume, grippe, maux de tête, douleur rhumatismale, douleur musculaire ou névralgie.
